**Europäisches Patentamt**

**European Patent Office**

**Office européen des brevets**

(19)

(11) Publication number: **0 116 439**
**A2**

(12)

# EUROPEAN PATENT APPLICATION

(21) Application number: **84300631.3**

(22) Date of filing: **01.02.84**

(51) Int. Cl.³: **A 61 K 7/06**

(30) Priority: **02.02.83 JP 14555/83**
**02.02.83 JP 14556/83**
**18.02.83 JP 24686/83**
**18.02.83 JP 24687/83**
**19.03.83 JP 45293/83**
**19.03.83 JP 45292/83**

(43) Date of publication of application:
**22.08.84 Bulletin 84/34**

(84) Designated Contracting States:
**AT BE CH DE FR GB IT LI LU NL SE**

(71) Applicant: **Suntory Limited**
**1-40 Dojimahama 2-chome Kita-ku**
**Osaka-shi Osaka-fu 530(JP)**

(72) Inventor: **Yoshizumi, Hajime**
**6-1-612, Kosobe-cho 2-chome**
**Takatsuki-shi Osaka(JP)**

(72) Inventor: **Amachi, Teruo**
**1-10, Hibarigaokayamate 2-chome**
**Takarazuka-shi Hyogo-ken(JP)**

(72) Inventor: **Kusumi, Takaaki**
**15-c-402, Yamate-cho 3-chome**
**Suita-shi Osaka(JP)**

(72) Inventor: **Tanaka, Takaharu**
**5-1-801, Higashiawaji-cho 1-chome**
**Higashi-yodogawa-ku Osaka-shi Osaka(JP)**

(72) Inventor: **Ishigooka, Hiroshi**
**4-5, Nishichujo-cho**
**Ibaraki-shi Osaka(JP)**

(74) Representative: **Ford, Michael Frederick et al,**
**MEWBURN ELLIS & CO. 2/3 Cursitor Street**
**London EC4A 1BQ(GB)**

(54) **Fatty acid containing hair tonic composition.**

(57) A hair tonic composition containing, as an effective ingredient, at least one compound selected from the group consisting of linoleic acid, oleic acid, arachidonic acid, linolenic acid, parinaric acid, petroselinic acid, palmitoleic acid, n-capric acid, n-caprylic acid, lauric acid, myristic acid and palmitic acid and the cosmetically acceptable salts therof. This hair tonic composition can effectively prevent the generation of dandruff and itching and can also effectively accelerate the growth of hair.

EP 0 116 439 A2

Croydon Printing Company Ltd.

## FATTY ACID CONTAINING
## HAIR TONIC COMPOSITION

The present invention relates to a novel hair tonic composition suitable for use in preventing the generation of dandruff (or scurf) in hair and itching in the scalp and in accelerating the growth of hair. More specifically, it relates to a novel hair tonic composition containing, as an effective ingredient, at least one fatty acid and/or the cosmetically acceptable salts thereof.

The possession of a healthy and profuse head of hair throughout the life is the ambition of most human beings. Various kinds of hair dressings, including hair tonic compositions, have been used for alleviating or curing epilation or depilation (i.e., the involuntary loss of hair and subsequent balding). However, although various kinds of disease appear, such as alopecia, the causes thereof and the mechanisms thereof are not fully understood. Accordingly, at present, there are no truly effective agents for alleviating the epilation, accelerating the growth of hair, and further alleviating or curing the generation of dandruff in the hair and itching in the scalp.

Accordingly, an object of the present invention is to provide a novel hair tonic composition capable of effectively depressing the generation of dandruff and itching in the hair and scalp and also of exhibiting a remarkable and real acceleration of the growth of hair.
Other objects and advantages of the present invention will be apparent from the description set forth hereinbelow.

In accordance with the present invention, there is provided a hair tonic composition containing, as an effective ingredient, at least one compound selected from the group consisting of linoleic acid, oleic acid, arachidonic acid, linolenic acid, parinaric acid, petroselinic acid, palmitoleic acid, n-capric acid, n-caprylic acid, lauric acid, myristic acid and palmitic acid and the cosmetically acceptable salts thereof.

The inventors have previously found that, after making a detailed comparative study of the microflora of the scalps of persons with healthy hair and those of persons with unhealthy hair (e.g., the generation of dandruff and itching in the scalp and abnormal depilation), Staphylococcus capitis constitutes all or most parts of the microflora in the scalp of those with healthy hair, and that, when the cells of Staphylococcus capitis or the treated products thereof are applied to the scalp, the remarkable growth effect of hair can be obtained. Furthermore, the inventors have clarified that the cells of Staphylococcus capitis have a lipase activity and testosterone 5α-reductase (i.e., "5α-reductase") inhibitory activity. The term "5α-reductase" denotes an enzyme which reduces testosterone to 5α-dihydrotestosterone. Based on these findings, the inventors have suggested that the above-mentioned activities are closely correlated with the growth effect of hair (see U.S. Patent Application No. 505,273 or European Patent Application No. 83303654.4).

The inventors have also found that, when the correlation of the above-mentioned lipase activity and 5α-reductase inhibitory activity with the hair growth effect is studied in detail, products containing fatty acids, as a main constituent, obtained from the interaction of lipase from Staphylococcus capitis with fats and oils, have a strong 5α-reductase inhibitory activity.

The inventors have surprisingly found that, after making a detailed study of the active component in the above--mentioned fatty acids, linoleic acid, oleic acid, arachidonic acid, linolenic acid, parinaric acid, petroselinic acid, palmitoleic acid, n-capric acid, n-caprylic acid, lauric acid, myristic acid or palmitic acid or the salts thereof have a strong 5α-reductase inhibitory activity and the fatty acids per se have an effective action on the growth of hair. As a result, the present invention has been completed.

Japanese Unexamined Patent Publication (Kokai) No. 56-61308 discloses a hair treatment composition for alleviating the generation of dandruff in the hair and itching in the scalp. This composition contains zinc pyrithione and certain carboxylic acid compounds. However, there is no description relating to the fatty acids of the present invention in this publication. Furthermore, this publication only discloses that the specified carboxylic acid compounds have a very gentle or moderate action to the human bodies and cause no pathogenic irritation against the eyes and the skins. Neither the 5α-reductase inhibitory activity of the fatty acids nor the hair growth acceleration effect thereof is taught in this publication.

Japanese National Publication of International Application (Kohyo) 57-502166 discloses a hair treatment containing betaine and certain aliphatic organic acids. However, there is no description relating to the fatty acids of the present invention in this publication. Furthermore, this publication only discloses that this hair treatment is effective for affording a good brushing property to the hair without causing any physical damages on the hair. There is no description relating to the growth effect of hair in this publication at all.

The active ingredients used in the hair tonic composition of the present invention are linoleic (or linolic) acid, oleic acid, arachidonic acid, linolenic acid, parinaric acid, petroselinic acid, palmitoleic acid, n-capric acid, n-caprylic acid, lauric acid, myristic acid and palmitic acid per se and the cosmetically acceptable various salts thereof. Examples of the cosmetically acceptable salts are inorganic salts such as an ammonium salt, alkali metal salts (e.g., lithium salt, potassium salt, sodium salt), alkaline earth metal salts (e.g., magnesium salt), and an aluminum salt. These active ingredients can be used alone or in any mixture thereof.

The amounts of the active ingredients incorporated into the hair tonic compositions according to the present invention are not particularly limited to the specified range, since the active ingredients have no pathogenic activity to human bodies. Thus, although the amount of the active ingredient incorporated into the hair tonic composition can be varied in a wide range, the preferable amount is 0.01% to 10% by weight, based on the total amount of the hair tonic composition. When two or more ingredients are used, the total amount thereof is preferably 0.01% to 10% by weight based on the total amount of the hair tonic composition. When the amount of the active ingredient is less than 0.01% by weight, the desirable growth effect of hair is not fully obtained. Contrary to this, when the amount of the active ingredient is more than 10% by weight, the desireble growth effect of hair is saturated and, and it is used, feeling is unconfortable.

The base materials used in the hair tonic compositions according to the present invention can include those conventionally used in any cosmetic compositions. Examples of such base materials which can be used in the present invention are distilled water; monohydric alcohols such as ethyl alcohol; polyhydric alcohols such as glycerine and ethylene glycol; fats and oils; surfactants.

In addition to the above-mentioned active ingredients, various conventional ingredients suitably used in the formulation of a hair tonic composition or a hair dressing composition can be incorporated in a conventional amount into the hair tonic composition of the present invention. Typical examples of such ingredients are hormones, vitamins, amino acids, herb extracts, photosensitizing dyes, resorcinol, menthol, wetting agents, and perfumes. These ingredients can be optionally incorporated into the hair tonic composition of the present invention unless the desired effect of the present invention is impaired. Especially, since unsaturated fatty acids such as linoleic acid, parinaric acid, palmitoleic acid, petroselinic acid, arachidone acid, and oleic acid are susceptible to oxidation, an antioxidant such as tocopherol can be preferably incorporated into the present hair tonic composition in an amount of, for example, one to one-tenth time of the fatty acid.

The final forms of the hair tonic composition according to the present invention can be any conventional form of hair tonic or hair dressing compositions such as hair tonic, hair lotion, hair cream, shampoo, linse, hair liquid, hair oil, pomade, and hair stick. Other forms can also be utilized.

The hair growth effect of the active ingredients used in the present invention is not clearly understood, but an attempted explanation is given hereinbelow without prejudice to the present invention.

Various theories have been proposed relating to the causes of depilation, epilation, dandruff, and itching. For example, an unbalanced hormone constitution theory, a nutrient relating theory, a seborrhea theory, and a genetic or hereditary theory are known; and it appears that there is a high correlation between the above-mentioned abnormal conditions and the development of sebaceous gland (see Masumi Inaba, "Mainichi Life" November, 1981, pages 26 to 35; "Saishin Keshohin Kagaku (Recent Cosmetics Science)", pages 130 published by Yakuji Nippo Sha in 1980; Kenji Adachi et. al., "Biochemical and Biophysical Research Communication, 41 (4), pages 884 to 890 (1970); Susumu Takayasu et. al., Journal of Investigative Dermatology 74, pages 187 to 191, 1980).

That is, when the sebaceous gland of the scalp is developed by nutrients, hormones or the like, the amount of 5α-reductase in the sebaceous gland increases. The level of 5α-dihydrotestosterone, more active androgen, becomes high. This tissue active androgen is transferred to hair papilla via blood vessels, thereby alleviating the activities of adenylcyclase in hair-matrix cells. As a result, it is believed that the size of hair-follicles is gradually reduced, therefore, the hair becomes thin and downy. Years of repetition of these processes cause baldness.

Based on these mechanisms for generating depilation and epilation, the 5α-reductase inhibitory activity is an important characteristic and property, which the hair tonic composition should have, and also become a standard or criterion for scientifically evaluating the effect of the hair tonic or hair dressing compositions.

As mentioned above, the fatty acids and the salts thereof used in the present invention have a strong 5α-reductase inhibitory activity and the strong hair growth effect of the present hair tonic composition is believed to be caused by the above-mentioned mechanism due to the 5α-reductase inhibitory activity. Furthermore, the microflora of the scalp is maintained in or brought to a healthy state by the fatty acids and the salts thereof contained in the hair tonic composition according to the present invention. It is considered that when these actions or functions are combined or multiplied they exhibit a strong hair growth acceleration effect.

The hair tonic composition according to the present invention has the 5α-reductase inhibitory activity as mentioned above, and when the hair tonic composition according to the present invention is applied to the human scalp or animal skins, strong hair growth acceleration effects can be provided. That is, when the hair tonic composition according to the present invention is applied to the human scalp, the depilation and epilation can be effectively alleviated, downy hairs become healthy, and the generation of dandruff and itching can be prevented. Furthermore, when the hair tonic composition of the present invention is applied to animals, the growth velocity of the hair (fur) remarkably increases.

The nonpathogenicity of the hair tonic composition according to the present invention has been confirmed. That is, the fatty acids of the present invention and the salts thereof were independently dispersed in an ethanol, and the mixtures were spread on the skins of five rabbits once a day for 3 days in each mixture. The ethanol was also spread in the same manner as a control. As a result, no abnormal condition was found in each case.

The 5α-reductase inhibitory activity was determined as follows.

Prostate gland cells of rats were crushed and a specimen of testosterone 5α-reductase was then prepared by separating microsome from the crushed liquid mixture. The conversion of the testosterone to 5α-dihydrotestosterone by the use of the above-prepared enzyme specimen was monitored by radioisotopically labelled testosterone. The reaction mixture was extracted with ethyl acetate and the extract was developed twice by silica gel thin layer chromatography (solvent system, dichloromethane: cyclohexane:acetone = 15:4:1). The amount of 5α-dihydrotestosterone were determined from the intensities of the radioactivity.

Reaction

A 30 µl amount of a 0.05 M phosphate buffer
(pH = 6.6), 10 µl of an enzyme specimen, 8.5 pmol of
labelled testosterone, 50 nmol of a reduced form of NADP
(nicotinamide adenine dinucleotide phosphate), and 10 µl
of a test sample were mixed (final volume = 50 µl). The
mixture was included at a temperature of 25°C for
60 minutes.

The reaction was stopped by the addition of 100 µl
of ethyl acetate, and the reaction mixture was extracted
by vigorous shaking. The extract was developed in the
same manner as mentioned above and the intensity of the
radioisotope was measured by using a scintillation
counter.

The above-mentioned determination was applied to
the samples having various concentrations. The 5α-
-reductase inhibitory activity was obtained as a inhibitory rate (%) or a 50% inhibitory
concentration ($IC_{50}$).

The 5α-reductase inhibitiory activity $IC_{50}$ of the
fatty acids of the present invention are as follows:

| Fatty Acid | $IC_{50}$ (mM) |
|---|---|
| Linoleic acid | 0.18 |
| Olic acid | 0.32 |
| Arachidonic acid | 0.15 |
| Linolenic acid | 1.6 |
| Parinaric acid | 1.1 |
| Petroselinic acid | 0.28 |
| Palmitoleic acid | 0.18 |
| n-Capric acid | 0.5 |
| n-Caprylic acid | 1.7 |
| Lauric acid | 0.6 |
| Myristic acid | 1.8 |
| Palmitic acid | 0.3 |

EXAMPLES

The present invention will now be further illustrated by, but is by no means limited to, the following examples, in which the preparation, application, and effect of the hair tonic composition of the present invention are specifically disclosed.

Example 1 (Formulation of Hair Tonic Composition)

The following hair tonic compositions were formulated:

(1) Hair Tonic type:

| Ingredient | Content (wt%) |
|---|---|
| 95% Ethyl alcohol | 50 |
| Castor oil | 2 |
| Perfume | 0.5 |
| Linoleic acid | 0.05 |
| Distilled water | to 100 |

(2) Hair Lotion type:

| Ingredient | Content (wt%) |
|---|---|
| Stearic acid | 5 |
| Cetyl alcohol | 0.5 |
| Emulsifier (polyoxyethylene sorbitan sesquioleate) | 1 |
| Glycerine | 5 |
| 95% Ethyl alcohol | 10 |
| Tocopherol | 0.1 |
| Perfume | 1 |
| Sodium linoleate | 0.5 |
| Distilled water | to 100 |

(3) Hair Cream type:

| Ingredient | Content (wt%) |
|---|---|
| Liquid paraffin | 33 |
| Bees wax | 5 |
| Lanolin | 3 |
| Vaseline | 5 |
| Emulsifier (polyoxyethylene sorbitan sesquioleate) | 2 |
| Ammonium linoleate | 0.5 |
| Polyethylene glycol | 1 |
| Tocopherol | 0.1 |
| Distilled water | to 100 |

(4) Shampoo type:

| Ingredient | Content (wt%) |
|---|---|
| Sodium alkyl ether sulfate | 16 |
| Lauric acid diethanolamide | 4 |
| Propylene glycol | 2 |
| Cetyl alcohol | 1 |
| Preservative, Dye, and Perfume | 1 |
| Linoleic acid | 0.05 |
| Arachidonic acid | 0.05 |
| Distilled water | to 100 |

(5) Aerosol type:

| Ingredient | Content (wt%) |
|---|---|
| Polyoxyethylene lanolin | 1.0 |
| Lanolin alcohol | 2.5 |
| Glycerine fatty acid ester | 0.5 |
| Perfume | 0.2 |
| Linoleic acid | 0.05 |
| Denatured alcohol (absolute) | 25.75 |
| Propellant Freon 12/11 40/60 | 70 |

(6) Pomade type:

| Ingredient | Content (wt%) |
|---|---|
| Japanese wax | 12 |
| Castor oil | 84.6 |
| Hardened oil | 2 |
| Tocopherol | 0.2 |
| Linoleic acid | 1.0 |
| Perfume | 0.2 |

(7) Hair Tonic type:

| Ingredient | Content (wt%) |
|---|---|
| 95% Ethyl alcohol | 50 |
| Castor oil | 2 |
| Tocopherol | 0.1 |
| Perfume | 0.5 |
| Oleic acid | 0.2 |
| Distilled water | to 100 |

(8) Hair Lotion type:

| Ingredient | Content (wt%) |
|---|---|

| | |
|---|---|
| Stearic acid | 5 |
| Cetyl alcohol | 0.5 |
| Emulsifier (polyoxyethylene sorbitan sesquioleate) | 1 |
| Glycerine | 5 |
| 95% Ethyl alcohol | 10 |
| Tocopherol | 0.1 |
| Perfume | 1 |
| Sodium oleate | 0.5 |
| Distilled water | to 100 |

(9) Hair Cream type:

| Ingredient | Content (wt%) |
|---|---|
| Liquid paraffin | 33 |
| Bees wax | 5 |
| Lanolin | 3 |
| Vaseline | 5 |
| Emulsifier (polyoxyethylene sorbitan sesquioleate) | 2 |
| Ammonium oleate | 0.5 |
| Polyethylene glycol | 1 |
| Tocopherol | 0.1 |
| Distilled water | to 100 |

(10) Shampoo type:

| Ingredient | Content (wt%) |
|---|---|
| Sodium alkyl ether sulfate | 16 |
| Lauric acid diethanolamide | 4 |
| Propylene glycol | 2 |
| Cetyl alcohol | 1 |
| Preservative, Dye, and Perfume | 1 |
| Oleic acid | 0.05 |
| Arachidonic acid | 0.05 |
| Distilled water | to 100 |

(11) Aerosol type:

| Ingredient | Content (wt%) |
|---|---|
| Polyoxyethylene lanolin | 1.0 |
| Lanolin alcohol | 2.5 |
| Glycerine fatty acid ester | 0.5 |

| Tocopherol | 0.1 |
|---|---|
| Perfume | 0.2 |
| Oleic acid | 0.05 |
| Denatured alcohol (absolute) | 25.65 |
| Propellant Freon 12/11 40/60 | 70 |

(12) Pomade type:

| Ingredient | Content (wt%) |
|---|---|
| Japanese wax | 12 |
| Castor oil | 84.6 |
| Hardened oil | 2 |
| Tocopherol | 0.2 |
| Oleic acid | 1.0 |
| Perfume | 0.2 |

(13) Hair Tonic type:

| Ingredient | Content (wt%) |
|---|---|
| 95% Ethyl alcohol | 50 |
| Castor oil | 2 |
| Tocopherol | 0.1 |
| Perfume | 0.5 |
| Arachidonic acid | 0.2 |
| Distilled water | to 100 |

(14) Hair Lotion type:

| Ingredient | Content (wt%) |
|---|---|
| Stearic acid | 5 |
| Cetyl alcohol | 0.5 |
| Emulsifier (polyoxyethylene sorbitan sesquioleate) | 1 |
| Glycerine | 5 |
| 95% Ethyl alcohol | 10 |
| Tocopherol | 0.1 |
| Perfume | 1 |
| Sodium arachidonate | 0.5 |
| Distilled water | to 100 |

(15) Hair Cream type:

| Ingredient | Content (wt%) |
|---|---|
| Liquid paraffin | 33 |
| Bees wax | 5 |

| Lanolin | 3 |
| Vaseline | 5 |
| Emulsifier (polyoxyethylene sorbitan sesquioleate) | 2 |
| Ammonium arachidonate | 0.5 |
| Polyethylene glycol | 1 |
| Tocopherol | 0.1 |
| Distilled water | to 100 |

(16) Shampoo type:

| Ingredient | Content (wt%) |
| --- | --- |
| Sodium alkyl ether sulfate | 16 |
| Lauric acid diethanolamide | 4 |
| Propylene glycol | 2 |
| Cetyl alcohol | 1 |
| Preservative, Dye, and Perfume | 1 |
| Linoleic acid | 0.05 |
| Arachidonic acid | 0.05 |
| Distilled water | to 100 |

(17) Aerosol type:

| Ingredient | Content (wt%) |
| --- | --- |
| Polyoxyethylene lanolin | 1.0 |
| Lanolin alcohol | 2.5 |
| Glycerine fatty acid ester | 0.5 |
| Tocopherol | 0.1 |
| Perfume | 0.2 |
| Arachidonic acid | 0.05 |
| Denatured alcohol (absolute) | 25.65 |
| Propellant Freon 12/11 40/60 | 70 |

(18) Pomade type:

| Ingredient | Content (wt%) |
| --- | --- |
| Japanese wax | 12 |
| Castor oil | 84.6 |
| Hardened oil | 2 |
| Tocopherol | 0.2 |
| Arachidonic acid | 1.0 |
| Perfume | 0.2 |

(19) Hair Tonic type:

0116439

- 14 -

| Ingredient | Content (wt%) |
| --- | --- |
| 95% Ethyl alcohol | 50 |
| Castor oil | 2 |
| Perfume | 0.5 |
| Tocopherol | 0.1 |
| Linolenic acid | 0.1 |
| Distilled water | to 100 |

(20) Hair Lotion type:

| Ingredient | Content (wt%) |
| --- | --- |
| Emulsifier (polyoxyethylene sorbitan sesquioleate) | 1 |
| Glycerine | 5 |
| 95% Ethyl alcohol | 10 |
| Tocopherol | 0.1 |
| Perfume | 1 |
| Sodium linolenate | 1 |
| Distilled water | to 100 |

(21) Hair Cream type:

| Ingredient | Content (wt%) |
| --- | --- |
| Liquid paraffin | 33 |
| Bees wax | 5 |
| Lanolin | 3 |
| Vaseline | 5· |
| Emulsifier (polyoxyethylene sorbitan sesquioleate) | 2 |
| Ammonium linolenate | 0.5 |
| Polyethylene glycol | 1 |
| Tocopherol | 0.1 |
| Distilled water | to 100 |

(22) Shampoo type:

| Ingredient | Content (wt%) |
| --- | --- |
| Sodium alkyl ether sulfate | 16 |
| Lauric acid diethanolamide | 4 |
| Propylene glycol | 2 |
| Cetyl alcohol | 1 |
| Preservative, Dye, and Perfume | 1 |
| Linolenic acid | 0.05 |

Arachidonic acid        0.05

Distilled water        to 100

(23) Aerosol type:

| Ingredient | Content (wt%) |
| --- | --- |
| Polyoxyethylene lanolin | 1.0 |
| Lanolin alcohol | 2.5 |
| Glycerine fatty acid ester | 0.5 |
| Tocopherol | 0.1 |
| Perfume | 0.2 |
| Linolenic acid | 0.05 |
| Denatured alcohol (absolute) | 25.65 |
| Propellant Freon 12/11 40/60 | 70 |

(24) Pomade type:

| Ingredient | Content (wt%) |
| --- | --- |
| Japanese wax | 12 |
| Castor oil | 80.6 |
| Hardened oil | 2 |
| Tocopherol | 0.2 |
| Linolenic acid | 5.0 |
| Perfume | 0.2 |

(25) Hair Tonic type:

| Ingredient | Content (wt%) |
| --- | --- |
| 95% Ethyl alcohol | 50 |
| Castor oil | 2 |
| Perfume | 0.5 |
| Parinaric acid | 0.05 |
| Distilled water | to 100 |

(26) Hair Lotion type:

| Ingredient | Content (wt%) |
| --- | --- |
| Emulsifier (polyoxyethylene sorbitan sesquioleate) | 1 |
| Glycerine | 5 |
| 95% Ethyl alcohol | 10 |
| Tocopherol | 0.1 |
| Perfume | 1 |
| Sodium parinarate | 0.5 |
| Distilled water | to 100 |

(27) Hair Cream type:

| Ingredient | Content (wt%) |
|---|---|
| Liquid paraffin | 33 |
| Bees wax | 5 |
| Lanolin | 3 |
| Vaseline | 5 |
| Emulsifier (polyoxyethylene sorbitan sesquioleate) | 2 |
| Ammonium parinarate | 0.5 |
| Polyethylene glycol | 1 |
| Tocopherol | 0.1 |
| Distilled water | to 100 |

(28) Shampoo type:

| Ingredient | Content (wt%) |
|---|---|
| Sodium alkyl ether sulfate | 16 |
| Lauric acid diethanolamide | 4 |
| Propylene glycol | 2 |
| Cetyl alcohol | 1 |
| Preservative, Dye, and Perfume | 1 |
| Parinaric acid | 0.1 |
| Linolic acid | 0.05 |
| Distilled water | to 100 |

(29) Aerosol type:

| Ingredient | Content (wt%) |
|---|---|
| Polyoxyethylene lanolin | 1.0 |
| Lanolin alcohol | 2.5 |
| Glycerine fatty acid ester | 0.5 |
| Tocopherol | 0.1 |
| Perfume | 0.2 |
| Parinaric acid | 0.1 |
| Denatured alcohol (absolute) | 25.6 |
| Propellant Freon 12/11 40/60 | 70 |

(30) Pomade type:

| Ingredient | Content (wt%) |
|---|---|
| Japanese wax | 12 |
| Castor oil | 84.6 |
| Hardened oil | 2 |

| | |
|---|---|
| Tocopherol | 0.2 |
| Parinaric acid | 1.0 |
| Perfume | 0.2 |

(31) Hair Tonic type:

| Ingredient | Content (wt%) |
|---|---|
| 95% Ethyl alcohol | 50 |
| Castor oil | 2 |
| Tocopherol | 0.05 |
| Perfume | 0.5 |
| Petroselinic acid | 0.05 |
| Distilled water | to 100 |

(32) Hair Lotion type:

| Ingredient | Content (wt%) |
|---|---|
| Emulsifier (polyoxyethylene sorbitan sesquioleate) | 1 |
| Glycerine | 5 |
| 95% Ethyl alcohol | 10 |
| Tocopherol | 0.1 |
| Perfume | 1 |
| Sodium petroselinate | 1 |
| Distilled water | to 100 |

(33) Hair Cream type:

| Ingredient | Content (wt%) |
|---|---|
| Liquid paraffin | 33 |
| Bees wax | 5 |
| Lanolin | 3 |
| Vaseline | 3 |
| Emulsifier (polyoxyethylene sorbitan sesquioleate) | 2 |
| Ammonium petroselinate | 0.5 |
| Polyethylene glycol | 1 |
| Tocopherol | 0.1 |
| Distilled water | to 100 |

(34) Shampoo type:

| Ingredient | Content (wt%) |
|---|---|
| Sodium alkyl ether sulfate | 16 |
| Lauric acid diethanolamide | 4 |
| Propylene glycol | 2 |

| Cetyl alcohol | 1 |
|---|---|
| Preservative, Dye, and Perfume | 1 |
| Petroselinic acid | 0.5 |
| Stearic acid | 0.05 |
| Distilled water | to 100 |

(35) Aerosol type:

| Ingredient | Content (wt%) |
|---|---|
| Polyoxyethylene lanolin | 1.0 |
| Lanolin alcohol | 2.5 |
| Glycerine fatty acid ester | 0.5 |
| Tocopherol | 0.1 |
| Perfume | 0.2 |
| Petroselinic acid | 0.1 |
| Denatured alcohol (absolute) | 25.6 |
| Propellant Freon 12/11 40/60 | 70 |

(36) Pomade type:

| Ingredient | Content (wt%) |
|---|---|
| Japanese wax | 12 |
| Castor oil | 84.6 |
| Hardened oil | 2 |
| Tocopherol | 0.2 |
| Petroselinic acid | 1.0 |
| Perfume | 0.2 |

(37) Hair Tonic type:

| Ingredient | Content (wt%) |
|---|---|
| 95% Ethyl alcohol | 50 |
| Castor oil | 2 |
| Tocopherol | 0.1 |
| Perfume | 0.5 |
| Palmitoleic acid | 0.1 |
| Distilled water | to 100 |

(38) Hair Lotion type:

| Ingredient | Content (wt%) |
|---|---|
| Emulsifier (polyoxyethylene sorbitan sesquioleate) | 1 |
| Glycerine | 5 |
| 95% Ethyl alcohol | 10 |

| | | |
|---|---|---|
| | Tocopherol | 0.1 |
| | Perfume | 1 |
| | Sodium palmitoleate | 0.5 |
| | Distilled water | to 100 |

(39) Hair Cream type:

| Ingredient | Content (wt%) |
|---|---|
| Liquid paraffin | 33 |
| Bees wax | 5 |
| Lanolin | 3 |
| Vaseline | 5 |
| Emulsifier (polyoxyethylene sorbitan sesquioleate) | 2 |
| Ammonium palmitoleate | 0.5 |
| Polyethylene glycol | 1 |
| Tocopherol | 0.1 |
| Distilled water | to 100 |

(40) Shampoo type:

| Ingredient | Content (wt%) |
|---|---|
| Sodium alkyl ether sulfate | 16 |
| Lauric acid diethanolamide | 4 |
| Propylene glycol | 2 |
| Cetyl alcohol | 1 |
| Preservative, Dye, and Perfume | 1 |
| Palmitoleic acid | 0.05 |
| Linolic acid | 0.05 |
| Distilled water | to 100 |

(41) Aerosol type:

| Ingredient | Content (wt%) |
|---|---|
| Polyoxyethylene lanolin | 1.0 |
| Lanolin alcohol | 2.5 |
| Glycerine fatty acid ester | 0.5 |
| Perfume | 0.2 |
| Palmitoleic acid | 0.05 |
| Denatured alcohol (absolute) | 25.75 |
| Propellant Freon 12/11 40/60 | 70 |

(42) Pomade type:

| Ingredient | Content (wt%) |
|---|---|

| Japanese wax | 12 |
|---|---|
| Castor oil | 84.6 |
| Hardened oil | 2 |
| Tocopherol | 0.2 |
| Palmitoleic acid | 0.5 |
| Perfume | 0.7 |

(43) Hair Tonic type:

| Ingredient | Content (wt%) |
|---|---|
| 95% Ethyl alcohol | 50 |
| Castor oil | 2 |
| Perfume | 0.5 |
| n-Capric acid | 0.1 |
| Distilled water | to 100 |

(44) Hair Lotion type:

| Ingredient | Content (wt%) |
|---|---|
| Emulsifier (polyoxyethylene sorbitan sesquioleate) | 1 |
| Glycerine | 5 |
| 95% Ethyl alcohol | 10 |
| Perfume | 1 |
| Sodium n-caprate | 1 |
| Distilled water | to 100 |

(45) Hair Cream type:

| Ingredient | Content (wt%) |
|---|---|
| Liquid paraffin | 33 |
| Bees wax | 5 |
| Lanolin | 3 |
| Vaseline | 5 |
| Emulsifier (polyoxyethylene sorbitan sesquioleate) | 2 |
| Ammonium n-Caprate | 0.5 |
| Polyethylene glycol | 1 |
| Distilled water | to 100 |

(46) Shampoo type:

| Ingredient | Content (wt%) |
|---|---|
| Sodium alkyl ether sulfate | 16 |
| Lauric acid diethanolamide | 4 |

| | |
|---|---|
| Propylene glycol | 2 |
| Cetyl alcohol | 1 |
| Preservative, Dye, and Perfume | 1 |
| n-Capric acid | 0.5 |
| Arachidonic acid | 0.05 |
| Distilled water | to 100 |

(47) Aerosol type:

| Ingredient | Content (wt%) |
|---|---|
| Polyoxyethylene lanolin | 1.0 |
| Lanolin alcohol | 2.5 |
| Glycerine fatty acid ester | 0.5 |
| Perfume | 0.2 |
| n-Capric acid | 0.1 |
| Denatured alcohol (absolute) | 25.7 |
| Propellant Freon 12/11 40/60 | 70 |

(48) Pomade type:

| Ingredient | Content (wt%) |
|---|---|
| Japanese wax | 12 |
| Castor oil | 84.6 |
| Hardened oil | 2 |
| Tocopherol | 0.2 |
| n-Capric acid | 1.0 |
| Perfume | 0.2 |

(49) Hair Tonic type:

| Ingredient | Content (wt%) |
|---|---|
| 95% Ethyl alcohol | 50 |
| Castor oil | 2 |
| Perfume | 0.5 |
| n-Caprylic acid | 0.5 |
| Distilled water | to 100 |

(50) Hair Lotion type:

| Ingredient | Content (wt%) |
|---|---|
| Emulsifier (polyoxyethylene sorbitan sesquioleate) | 1 |
| Glycerine | 5 |
| 95% Ethyl alcohol | 10 |
| Perfume | 1 |

- 22 -

| | |
|---|---|
| Sodium n-caprylate | 1 |
| Distilled water | to 100 |

(51) Hair Cream type:

| Ingredient | Content (wt%) |
|---|---|
| Liquid paraffin | 33 |
| Bees wax | 5 |
| Lanolin | 3 |
| Vaseline | 5 |
| Emulsifier (polyoxyethylene sorbitan sesquioleate) | 2 |
| Ammonium n-caprylate | 1 |
| Polyethylene glycol | 1 |
| Tocopherol | 0.1 |
| Distilled water | to 100 |

(52) Shampoo type:

| Ingredient | Content (wt%) |
|---|---|
| Sodium alkyl ether sulfate | 16 |
| Lauric acid diethanolamide | 4 |
| Propylene glycol | 2 |
| Cetyl alcohol | 1 |
| Preservative, Dye, and Perfume | 1 |
| n-Caprylic acid | 0.1 |
| Linolic acid | 0.05 |
| Tocopherol | 0.01 |
| Distilled water | to 100 |

(53) Aerosol type:

| Ingredient | Content (wt%) |
|---|---|
| Polyoxyethylene lanolin | 1.0 |
| Lanolin alcohol | 2.5 |
| Glycerine fatty acid ester | 0.5 |
| Perfume | 0.2 |
| n-Caprylic acid | 0.5 |
| Denatured alcohol (absolute) | 25.3 |
| Propellant Freon 12/11 40/60 | 70 |

(54) Pomade type:

| Ingredient | Content (wt%) |
|---|---|
| Japanese wax | 12 |

| | |
|---|---|
| Castor oil | 84.6 |
| Hardened oil | 2 |
| Tocopherol | 0.2 |
| n-Caprylic acid | 1.0 |
| Perfume | 0.2 |

(55) Hair Tonic type:

| Ingredient | Content (wt%) |
|---|---|
| 95% Ethyl alcohol | 50 |
| Castor oil | 2 |
| Perfume | 0.5 |
| Lauric acid | 0.05 |
| Distilled water | to 100 |

(56) Hair Lotion type:

| Ingredient | Content (wt%) |
|---|---|
| Emulsifier (polyoxyethylene sorbitan sesquioleate) | 1 |
| Glycerine | 5 |
| 95% Ethyl alcohol | 10 |
| Tocopherol | 0.1 |
| Perfume | 1 |
| Sodium laurate | 0.5 |
| Distilled water | to 100 |

(57) Hair Cream type:

| Ingredient | Content (wt%) |
|---|---|
| Liquid paraffin | 33 |
| Bees wax | 5 |
| Lanolin | 3 |
| Vaseline | 5 |
| Emulsifier (polyoxyethylene sorbitan sesquioleate) | 2 |
| Ammonium laurate | 0.5 |
| Polyethylene glycol | 1 |
| Distilled water | to 100 |

(58) Shampoo type:

| Ingredient | Content (wt%) |
|---|---|
| Sodium alkyl ether sulfate | 16 |
| Lauric acid diethanolamide | 4 |

| | |
|---|---|
| Propylene glycol | 2 |
| Cetyl alcohol | 1 |
| Preservative, Dye, and Perfume | 1 |
| Lauric acid | 0.05 |
| Oleic acid | 0.05 |
| Distilled water | to 100 |

(59) Aerosol type:

| Ingredient | Content (wt%) |
|---|---|
| Polyoxyethylene lanolin | 1.0 |
| Lanolin alcohol | 2.5 |
| Glycerine fatty acid ester | 0.5 |
| Perfume | 0.2 |
| Lauric acid | 0.5 |
| Denatured alcohol (absolute) | 25.3 |
| Propellant Freon 12/11 40/60 | 70 |

(60) Pomade type:

| Ingredient | Content (wt%) |
|---|---|
| Japanese wax | 12 |
| Castor oil | 84.6 |
| Hardened oil | 2 |
| Tocopherol | 0.2 |
| Lauric acid | 0.5 |
| Linolic acid | 0.5 |
| Perfume | 0.2 |

(61) Hair Tonic type:

| Ingredient | Content (wt%) |
|---|---|
| 95% Ethyl alcohol | 50 |
| Castor oil | 2 |
| Perfume | 0.5 |
| Myristic acid | 0.05 |
| Distilled water | to 100 |

(62) Hair Lotion type:

| Ingredient | Content (wt%) |
|---|---|
| Emulsifier (polyoxyethylene sorbitan sesquioleate) | 1 |
| Glycerine | 5 |
| 95% Ethyl alcohol | 10 |

| | |
|---|---|
| Tocopherol | 0.1 |
| Perfume | 1 |
| Sodium myristate | 0.5 |
| Linolenic acid | 0.1 |
| Distilled water | to 100 |

(63) Hair Cream type:

| Ingredient | Content (wt%) |
|---|---|
| Liquid paraffin | 33 |
| Bees wax | 5 |
| Lanolin | 3 |
| Vaseline | 5 |
| Emulsifier (polyoxyethylene sorbitan sesquioleate) | 2 |
| Ammonium myristate | 1 |
| Polyethylene glycol | 1 |
| Distilled water | to 100 |

(64) Shampoo type:

| Ingredient | Content (wt%) |
|---|---|
| Sodium alkyl ether sulfate | 16 |
| Lauric acid diethanolamide | 4 |
| Propylene glycol | 2 |
| Cetyl alcohol | 1 |
| Preservative, Dye, and Perfume | 1 |
| Myristic acid | 0.5 |
| Oleic acid | 0.05 |
| Distilled water | to 100 |

(65) Aerosol type:

| Ingredient | Content (wt%) |
|---|---|
| Polyoxyethylene lanolin | 1.0 |
| Lanolin alcohol | 2.5 |
| Glycerine fatty acid ester | 0.5 |
| Perfume | 0.2 |
| Myristic acid | 0.1 |
| Denatured alcohol (absolute) | 25.7 |
| Propellant Freon 12/11 40/60 | 70 |

(66) Pomade type:

| Ingredient | Content (wt%) |
|---|---|

| Japanese wax | 12 |
|---|---|
| Castor oil | 84.6 |
| Hardened oil | 2 |
| Tocopherol | 0.2 |
| Myristic acid | 0.6 |
| Linolic acid | 0.4 |
| Perfume | 0.2 |

(67) Hair Tonic type:

| Ingredient | Content (wt%) |
|---|---|
| 95% Ethyl alcohol | 50 |
| Castor oil | 2 |
| Perfume | 0.5 |
| Palmitic acid | 0.05 |
| Distilled water | to 100 |

(68) Hair Lotion type:

| Ingredient | Content (wt%) |
|---|---|
| Emulsifier (polyoxyethylene sorbitan sesquioleate) | 1 |
| Glycerine | 5 |
| 95% Ethyl alcohol | 10 |
| Tocopherol | 0.1 |
| Perfume | 1 |
| Sodium palmitate | 0.5 |
| Distilled water | to 100 |

(69) Hair Cream type:

| Ingredient | Content (wt%) |
|---|---|
| Liquid paraffin | 33 |
| Bees wax | 5 |
| Lanolin | 3 |
| Vaseline | 5 |
| Emulsifier (polyoxyethylene sorbitan sesquioleate) | 2 |
| Ammonium palmitate | 0.5 |
| Polyethylene glycol | 1 |
| Distilled water | to 100 |

(70) Shampoo type:

| Ingredient | Content (wt%) |
|---|---|

| Sodium alkyl ether sulfate | 16 |
|---|---|
| Lauric acid diethanolamide | 4 |
| Propylene glycol | 2 |
| Cetyl alcohol | 1 |
| Preservative, Dye, and Perfume | 1 |
| Linoleic acid | 0.05 |
| Palmitic acid | 0.05 |
| Distilled water | to 100 |

(71) Aerosol type:

| Ingredient | Content (wt%) |
|---|---|
| Polyoxyethylene lanolin | 1.0 |
| Lanolin alcohol | 2.5 |
| Glycerine fatty acid ester | 0.5 |
| Perfume | 0.2 |
| Palmitic acid | 0.05 |
| Denatured alcohol (absolute) | 25.75 |
| Propellant Freon 12/11 40/60 | 70 |

Example 2 (Application of Hair Tonic Composition)

The hair tonic type compositions prepared in Example 1 were applied, twice a day, to the scalps of 10 men, each suffering from a large degree of dandruff and depilaton at ages of 28 to 40, in an amount of 2 to 4 ml a day for 3 months.

The results are as follows:

## Table

### Hair Tonic Type (1)

| Condition | Effect | | |
|---|---|---|---|
| | Excellent | Good | None |
| Dandruff | 6 | 2 | 2 |
| Depilation | 5 | 4 | 1 |

### Hair Tonic Type (7)

| Condition | Effect | | |
|---|---|---|---|
| | Excellent | Good | None |
| Dandruff | 4 | 5 | 1 |
| Itch | 10 | 0 | 0 |
| Depilation | 0 | 9 | 1 |

### Hair Tonic Type (13)

| Condition | Effect | | |
|---|---|---|---|
| | Excellent | Good | None |
| Dandruff | 6 | 3 | 1 |
| Itch | 6 | 3 | 1 |
| Depilation | 1 | 7 | 2 |

## Hair Tonic Type (19)

| Condition | Effect | | |
| --- | --- | --- | --- |
| | Excellent | Good | None |
| Dandruff | 1 | 8 | 1 |
| Itch | 9 | 1 | 0 |
| Depilation | 0 | 5 | 5 |

## Hair Tonic Type (25)

| Condition | Effect | | |
| --- | --- | --- | --- |
| | Excellent | Good | None |
| Dandruff | 2 | 7 | 1 |
| Itch | 8 | 1 | 1 |
| Depilation | 0 | 7 | 3 |

## Hair Tonic Type (31)

| Condition | Effect | | |
| --- | --- | --- | --- |
| | Excellent | Good | None |
| Dandruff | 5 | 3 | 2 |
| Itch | 9 | 1 | 0 |
| Depilation | 0 | 6 | 4 |

Hair Tonic Type (37)

| Condition | Effect | | |
|---|---|---|---|
| | Excellent | Good | None |
| Dandruff | 6 | 3 | 1 |
| Itch | 8 | 0 | 2 |
| Depilation | 0 | 9 | 1 |

Hair Tonic Type (43)

| Condition | Effect | | |
|---|---|---|---|
| | Excellent | Good | None |
| Dandruff | 3 | 5 | 2 |
| Itch | 7 | 1 | 2 |
| Depilation | 0 | 8 | 2 |

Hair Tonic Type (49)

| Condition | Effect | | |
|---|---|---|---|
| | Excellent | Good | None |
| Dandruff | 1 | 8 | 1 |
| Itch | 8 | 1 | 1 |
| Depilation | 0 | 8 | 2 |

### Hair Tonic Type (55)

| Condition | Effect | | |
|---|---|---|---|
| | Excellent | Good | None |
| Dandruff | 1 | 7 | 2 |
| Itch | 9 | 0 | 1 |
| Depilation | 0 | 7 | 3 |

### Hair Tonic Type (61)

| Condition | Effect | | |
|---|---|---|---|
| | Excellent | Good | None |
| Dandruff | 6 | 2 | 2 |
| Itch | 9 | 0 | 1 |
| Depilation | 0 | 9 | 1 |

### Hair Tonic Type (67)

| Condition | Effect | | |
|---|---|---|---|
| | Excellent | Good | None |
| Dandruff | 5 | 4 | 1 |
| Itch | 10 | 0 | 0 |
| Depilation | 0 | 9 | 1 |

Example 3 (Application to Rabbits)

Ten week old male rabbits were shorn on the back. A 0.2% fatty acid solution in 80% aqueous ethanol solution was applied, twice a day, to a half-side of the shorn portion of each rabbit for one week. As a control, a 80% aqueous ethanol solution was also applied, twice a day, to the other half-side of the shorn portion of each rabbit for one week (two week for linoleic acid). The length of the grown fur was measured.

The results are as follows:

Linoleic Acid

| Rabbit No. | Grown fur length | | Difference b |
|---|---|---|---|
| | Control side a | Test side a' | |
| 1 | 7.25 ± 0.87 | 7.83 ± 0.59 | 0.58 |
| 2 | 10.03 ± 0.26 | 10.25 ± 0.27 | 0.22 |
| 3 | 6.87 ± 0.40 | 7.21 ± 0.20 | 0.34 |
| 4 | 7.01 ± 0.25 | 7.36 ± 0.22 | 0.35 |
| 5 | 11.97 ± 0.35 | 12.51 ± 0.42 | 0.54 |
| 6 | 10.33 ± 0.26 | 10.78 ± 0.26 | 0.45 |
| 7 | 12.58 ± 0.73 | 13.13 ± 0.69 | 0.55 |
| 8 | 6.87 ± 0.36 | 7.26 ± 0.30 | 0.39 |
| 9 | 10.22 ± 0.45 | 10.68 ± 0.53 | 0.46 |
| 10 | 12.35 ± 0.63 | 12.73 ± 0.33 | 0.38 |
| Average | 9.55[*1] | 9.98[*2] | 0.43 |
| Standard error | − | − | 0.11 |

Oleic Acid

| Rabbit | Grown fur length | | Difference b |
| :---: | :---: | :---: | :---: |
| No. | Control side a | Test side a' | |
| 1 | 6.48 ± 0.28 | 7.53 ± 0.24 | 1.05 |
| 2 | 4.14 ± 0.12 | 4.62 ± 0.15 | 0.48 |
| 3 | 5.26 ± 0.26 | 5.91 ± 0.11 | 0.65 |
| 4 | 2.10 ± 0.17 | 2.63 ± 0.19 | 0.53 |
| 5 | 4.49 ± 0.20 | 5.03 ± 0.18 | 0.54 |
| 6 | 8.04 ± 0.13 | 8.75 ± 0.19 | 0.71 |
| 7 | 1.64 ± 0.13 | 2.09 ± 0.13 | 0.45 |
| 8 | 7.15 ± 0.25 | 7.71 ± 0.31 | 0.56 |
| 9 | 9.44 ± 0.27 | 10.29 ± 0.30 | 0.85 |
| 10 | 6.02 ± 0.16 | 6.46 ± 0.16 | 0.44 |
| Average | 5.476[*1] | 6.102[*2] | 0.626 |
| Standard error | − | − | 0.19 |

Arachidonic Acid

| Rabbit No. | Grown fur length | | Difference b |
|---|---|---|---|
| | Control side a | Test side a' | |
| 1 | 9.62 ± 0.28 | 11.01 ± 0.40 | 1.39 |
| 2 | 4.45 ± 0.23 | 5.22 ± 0.33 | 0.77 |
| 3 | 9.96 ± 0.22 | 10.67 ± 0.31 | 0.71 |
| 4 | 9.73 ± 0.19 | 10.47 ± 0.45 | 0.74 |
| 5 | 1.12 ± 0.10 | 1.51 ± 0.13 | 0.39 |
| 6 | 1.49 ± 0.08 | 1.79 ± 0.07 | 0.3 |
| 7 | 9.19 ± 0.23 | 9.77 ± 0.20 | 0.58 |
| 8 | 6.62 ± 0.28 | 7.36 ± 0.23 | 0.74 |
| 9 | 5.12 ± 0.20 | 5.92 ± 0.10 | 0.8 |
| 10 | 10.51 ± 0.55 | 11.89 ± 0.42 | 1.38 |
| Average | 6.781[*1] | 7.561[*2] | 0.78 |
| Standard error | − | − | 0.34 |

Linolenic Acid

| Rabbit No. | Grown fur length | | Difference b |
|---|---|---|---|
| | Control side a | Test side a' | |
| 1 | 1.53 ± 0.09 | 1.73 ± 0.08 | 0.20 |
| 2 | 7.19 ± 0.24 | 8.09 ± 0.13 | 0.90 |
| 3 | 5.10 ± 0.12 | 5.45 ± 0.13 | 0.35 |
| 4 | 9.17 ± 0.19 | 9.77 ± 0.30 | 0.60 |
| 5 | 2.08 ± 0.09 | 2.30 ± 0.08 | 0.22 |
| Average | 5.01[*1] | 5.46[*2] | 0.45 |
| Standard error | − | − | 0.26 |

Parinaric Acid

| Rabbit No. | Grown fur length | | Difference b |
|---|---|---|---|
| | Control side a | Test side a' | |
| 1 | 5.68 ± 0.10 | 6.20 ± 0.15 | 0.52 |
| 2 | 7.75 ± 0.14 | 8.76 ± 0.16 | 1.01 |
| 3 | 5.58 ± 0.14 | 5.69 ± 0.12 | 0.11 |
| 4 | 1.47 ± 0.09 | 1.54 ± 0.07 | 0.07 |
| 5 | 10.67 ± 0.37 | 11.96 ± 0.32 | 1.29 |
| Average | 6.23[*1] | 6.83[*2] | 0.60 |
| Standard error | - | - | 0.48 |

Petroselinic Acid

| Rabbit No. | Grown fur length | | Difference b |
|---|---|---|---|
| | Control side a | Test side a' | |
| 1 | $5.05 \pm 0.14$ | $5.35 \pm 0.09$ | 0.30 |
| 2 | $7.57 \pm 0.11$ | $8.49 \pm 0.18$ | 0.92 |
| 3 | $5.60 \pm 0.09$ | $5.80 \pm 0.08$ | 0.20 |
| 4 | $1.58 \pm 0.06$ | $1.72 \pm 0.07$ | 0.14 |
| 5 | $11.00 \pm 0.26$ | $11.95 \pm 0.40$ | 0.95 |
| Average | 6.16[*1] | 6.66[*2] | 0.50 |
| Standard error | − | − | 0.36 |

Palmitoleic Acid

| Rabbit No. | Grown fur length | | Difference b |
| --- | --- | --- | --- |
| | Control side a | Test side a' | |
| 1 | 13.13 ± 0.52 | 14.39 ± 0.47 | 1.26 |
| 2 | 4.37 ± 0.23 | 4.91 ± 0.18 | 0.54 |
| 3 | 10.3 ± 0.20 | 11.53 ± 0.22 | 1.23 |
| 4 | 9.47 ± 0.30 | 10.99 ± 0.28 | 1.52 |
| 5 | 1.32 ± 0.12 | 1.65 ± 0.09 | 0.33 |
| 6 | 1.54 ± 0.07 | 1.82 ± 0.07 | 0.28 |
| 7 | 8.76 ± 0.21 | 9.61 ± 0.13 | 0.85 |
| 8 | 6.75 ± 0.36 | 7.49 ± 0.24 | 0.74 |
| 9 | 5.05 ± 0.18 | 5.97 ± 0.21 | 0.92 |
| 10 | 11.20 ± 0.33 | 12.15 ± 0.17 | 0.95 |
| Average | 7.189[*1] | 8.051[*2] | 0.862 |
| Standard error | – | – | 0.38 |

n-Capric Acid

| Rabbit | Grown fur length | | Difference b |
|--------|------------------|-----------------|--------------|
| No. | Control side a | Test side a' | |
| 1 | $9.42 \pm 0.30$ | $10.34 \pm 0.18$ | 0.92 |
| 2 | $4.34 \pm 0.17$ | $4.48 \pm 0.12$ | 0.14 |
| 3 | $10.28 \pm 0.18$ | $10.95 \pm 0.22$ | 0.67 |
| 4 | $10.27 \pm 0.17$ | $10.96 \pm 0.24$ | 0.69 |
| 5 | $1.21 \pm 0.10$ | $1.51 \pm 0.83$ | 0.3 |
| 6 | $1.52 \pm 0.07$ | $1.82 \pm 0.08$ | 0.3 |
| 7 | $9.55 \pm 0.26$ | $10.15 \pm 0.14$ | 0.6 |
| 8 | $6.80 \pm 0.25$ | $7.39 \pm 0.10$ | 0.59 |
| 9 | $5.18 \pm 0.24$ | $5.75 \pm 0.16$ | 0.57 |
| 10 | $11.28 \pm 0.25$ | $12.07 \pm 0.18$ | 0.79 |
| Average | 6.985[1] | 7.542[2] | 0.557 |
| Standard error | − | − | 0.23 |

n-Caprylic Acid

| Rabbit No. | Grown fur length | | Difference b |
|:---:|:---:|:---:|:---:|
| | Control side a | Test side a' | |
| 1 | 1.57 ± 0.06 | 1.64 ± 0.10 | 0.07 |
| 2 | 6.97 ± 0.26 | 7.52 ± 0.14 | 0.55 |
| 3 | 5.59 ± 0.14 | 5.86 ± 0.12 | 0.27 |
| 4 | 9.07 ± 0.14 | 9.56 ± 0.08 | 0.49 |
| 5 | 2.09 ± 0.08 | 2.32 ± 0.07 | 0.23 |
| Average | 5.06[*1] | 5.38[*2] | 0.32 |
| Standard error | — | — | 0.18 |

Lauric Acid

| Rabbit | Grown fur length | | Difference b |
|--------|---------------|--------------|-----|
| No. | Control side a | Test side a' | |
| 1 | 1.35 ± 0.09 | 1.61 ± 0.07 | 0.26 |
| 2 | 6.06 ± 0.24 | 6.88 ± 0.44 | 0.82 |
| 3 | 6.52 ± 0.46 | 7.94 ± 0.45 | 1.42 |
| 4 | 6.34 ± 0.45 | 7.10 ± 0.26 | 0.76 |
| 5 | 4.2 ± 0.26 | 4.80 ± 0.22 | 0.6 |
| 6 | 1.94 ± 0.12 | 2.31 ± 0.14 | 0.37 |
| 7 | 1.46 ± 0.14 | 1.87 ± 0.18 | 0.41 |
| 8 | 8.80 ± 0.26 | 9.51 ± 0.35 | 0.71 |
| 9 | 6.48 ± 0.34 | 7.65 ± 0.13 | 1.17 |
| 10 | 5.76 ± 0.19 | 7.00 ± 0.26 | 1.24 |
| Average | 4.891[*1] | 5.667[*2] | 0.776 |
| Standard error | - | - | 0.37 |

**0116439**

Myristic Acid

| Rabbit No. | Grown fur length | | Difference b |
|---|---|---|---|
| | Control side a | Test side a' | |
| 1 | 6.37 ± 0.30 | 7.08 ± 0.24 | 0.71 |
| 2 | 4.19 ± 0.22 | 4.60 ± 0.23 | 0.41 |
| 3 | 10.19 ± 0.41 | 11.25 ± 0.38 | 1.06 |
| 4 | 2.07 ± 0.21 | 2.69 ± 0.20 | 0.62 |
| 5 | 4.51 ± 0.22 | 5.08 ± 0.13 | 0.57 |
| 6 | 8.09 ± 0.23 | 8.70 ± 0.15 | 0.61 |
| 7 | 1.49 ± 0.09 | 1.76 ± 0.09 | 0.27 |
| 8 | 7.11 ± 0.43 | 7.97 ± 0.25 | 0.86 |
| 9 | 9.37 ± 0.31 | 10.30 ± 0.35 | 0.93 |
| 10 | 5.83 ± 0.27 | 6.4 ± 0.24 | 0.57 |
| Average | 5.922[*1] | 6.583[*2] | 0.661 |
| Standard error | — | — | 0.22 |

Palmitic Acid

| Rabbit No. | Grown fur length | | Difference b |
| | Control side a | Test side a' | |
| --- | --- | --- | --- |
| 1 | 1.35 ± 0.14 | 1.71 ± 0.15 | 0.36 |
| 2 | 5.94 ± 0.38 | 7.18 ± 0.44 | 1.24 |
| 3 | 7.42 ± 0.49 | 8.98 ± 0.43 | 1.56 |
| 4 | 7.20 ± 0.45 | 8.80 ± 0.55 | 1.6 |
| 5 | 4.37 ± 0.29 | 5.04 ± 0.17 | 0.67 |
| 6 | 2.10 ± 0.13 | 2.56 ± 0.19 | 0.46 |
| 7 | 1.48 ± 0.14 | 1.98 ± 0.11 | 0.5 |
| 8 | 9.22 ± 0.23 | 10.37 ± 0.39 | 1.15 |
| 9 | 6.59 ± 0.34 | 7.28 ± 0.21 | 0.69 |
| 10 | 5.31 ± 0.37 | 6.66 ± 0.43 | 1.35 |
| Average | 5.098[*1] | 6.056[*2] | 0.958 |
| Standard error | – | – | 0.45 |

(Remarks in each table)

a:   Average fur growth length of control side
(mm) $\pm$ standard deviation

a':   Average fur growth length of test side
(mm) $\pm$ standard deviation

b:   Average fur growth length of test side -
average fur growth length of control side
(mm)

*1:   Mean value of average growth length of
control side

*2:   Mean value of average growth length of
test side

## CLAIMS

1. A hair tonic composition containing, as an effective ingredient, at least one compound selected from the group consisting of linoleic acid, oleic acid, arachidonic acid, linolenic acid, parinaric acid, petroselinic acid, palmitoleic acid, n-capric acid, n-caprylic acid, lauric acid, myristic acid and palmitic acid and the cosmetically acceptable salts thereof.

2. A hair tonic composition as claimed in claim 1, wherein the amount of said compound is 0.01% to 10% by weight based on the total weight of the composition.

3. A method for preparing a hair tonic composition comprising incorporating at least one compound selected from the group consisting of linoleic acid, oleic acid, arachidonic acid, linolenic acid, parinaric acid, petroselinic acid, palmitoleic acid, n-capric acid, n-caprylic acid, lauric acid, myristic acid and palmitic acid and the cosmetically acceptable salts thereof into the hair tonic composition.

4. A method for applying, to hair, a hair tonic composition containing, as an effective ingredient, at least one compound selected from the group consisting of linoleic acid, oleic acid, arachidonic acid, linolenic acid, parinaric acid, petroselinic acid, palmitoleic acid, n-capric acid, n-caprylic acid, lauric acid, myristic acid and palmitic acid and the cosmetically acceptable salts thereof.

5.    A process for the cosmetic treatment of the human scalp which comprises applying thereto a composition containing a base material which is physiologically acceptable for exterior use, together with at least one compound selected from linoleic acid, oleic acid, arachidonic acid, linolenic acid, parinaric acid, petroselinic acid, palmitoleic acid, n-capric acid, n-caprylic acid, lauric acid, myristic acid and palmitic acid and the cosmetically acceptable salts thereof.